(19) Europäisches Patentamt
European Patent Office
Office européen des brevets

(11) Veröffentlichungsnummer: **0 071 059**
**B1**

(12) # EUROPÄISCHE PATENTSCHRIFT

(45) Veröffentlichungstag der Patentschrift: **24.09.86**

(51) Int. Cl.⁴: **C 07 D 409/14,** A 61 K 31/50

(21) Anmeldenummer: **82106110.8**

(22) Anmeldetag: **08.07.82**

(54) **6-(5-(Omega-(1-Imidazolyl)-alkyl)-thien-2-yl)-3-oxo-2,3,4,5-tetrahydropyridazine und deren Säureadditionssalze, Verfahren zu ihrer Herstellung und diese enthaltende pharmazeutische Präparate.**

(30) Priorität: **31.07.81 DE 3130252**

(43) Veröffentlichungstag der Anmeldung: **09.02.83 Patentblatt 83/6**

(45) Bekanntmachung des Hinweises auf die Patenterteilung: **24.09.86 Patentblatt 86/39**

(84) Benannte Vertragsstaaten: **AT BE CH FR GB IT LI LU NL SE**

(56) Entgegenhaltungen:
**DE-A-2 151 216**
**GB-A-1 383 906**
**US-A-4 057 634**
**US-A-4 353 904**

**Chemical Abstracts Band 95, Nr. 7, 17. August 1981, Columbus, Ohio, USA J. BOURGUIGNON et al. "Pyridazines: 3-(thien-2-yl)pyridazine, some 6-substituted 3-(thien-2-yl)pyridazines and new condensed derivatives", Seite 713, Spalte 1, Abstract Nr. 62110f**

(73) Patentinhaber: **A. Nattermann & Cie. GmbH, Nattermannallee 1, D-5000 Köln 30 (DE)**

(72) Erfinder: **Lautenschläger, Hans- Heiner, Dr., Neusser Gasse 50, D-5024 Pulheim- Stommeln (DE)**
Erfinder: **Hilboll, Gerd, Dr., Dehmelstrasse 36, D-5000 Köln 30 (DE)**
Erfinder: **Friehe, Hugo, Dr., Am Burgfeld 94a, D-5042 Erftstadt- Lechenich (DE)**
Erfinder: **Löhr, Josef Peter, Dr., Henkenheide 55, D-4010 Hilden (DE)**

(74) Vertreter: **Redies, Bernd, Dr. rer. nat., COHAUSZ & FLORACK Patentanwaltsbüro Schumannstrasse 97 Postfach 14 01 47, D-4000 Düsseldorf 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 071 059 B1

LIBER, STOCKHOLM 1986

**Beschreibung**

Die Erfindung betrifft neue 6-{5-[ω-(1-Imidazolyl) -alkyl]-thien-2-yl}-3-oxo-2,3,4,5-tetrahydro-pyridazine, deren Säureadditionssalze, Verfahren zu ihrer Herstellung sowie ihre Verwendung als Wirkstoff in Arzneimitteln, insbesondere zur Behandlung von entzündlichen und thromboembolischen Erkrankungen. Die erfindungsgemäßen 6-{5-[ω -(1-Imidazolyl)-alkyl]-thien-2-yl}-3-oxo-2,3,4,5-tetrahydro-pyridazine entsprechen der allgemeinen Formel I

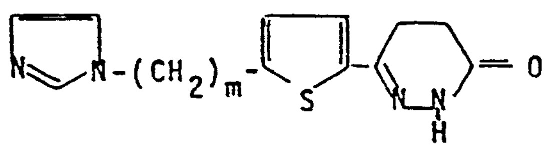

(I)

worin m eine ganze Zahl von 1-12, insbesondere 1-5, ist; umfaßt sind auch die Säureadditionssalze der Verbindungen der allgemeinen Formel I. Säureadditionssalze sind inbesondere pharmazeutisch verwendbare, untoxische Säureadditionssalze mit anorganischen Säuren, z.B. Chlorwasserstoffsäure, Bromwasserstoffsäure, Schwefelsäure oder Phosphorsäure, oder mit organischen Säuren, wie entsprechende Carbonsäuren. z.B. Essigsäure, Propionsäure, Oxalsäure, Malonsäure, Glykolsäure, Bernsteinsäure, Maleinsäure, Fumarsäure, Äpfelsäure, Weinsäure, Citronensäure, Benzoesäure und Zimtsäure.

In der GB-PS 1 383 906 sind 6-Phenyl-4,5-dihydro-3(2H)-pyridazinone mit verschiedenen Substituenten in verschiedenen Stellungen des Phenylrestes als antihypertensive Wirkstoffe beschrieben. In der DE-OS 2 151 216 ist ebenfalls ein p-substituiertes 6-Phenyl-4 5-dihydro-3(2H)-pyridazinon als Wirkstoff ohne nähere Angaben beschrieben. Zwar sind die erfindungsgemäßen Verbindungen in ihrer Grundstruktur 6-Thienyl-4,5-dihydro-3(2H)-pyridazon bekannt,nichts ist jedoch über ähnliche pharmakologische Eigenschaften offenbart.

Die Verbindungen der Erfindung weisen zusätzlich zu der blutdruckregulierenden Wirksamkeit der 6-substituierten 4,5-Dihydro-3(2H)-pyridazinone wertvolle pharmakologische Eigenschaften auf. Sie zeichnen sich einerseits durch eine starke Beeinflussung des Arachidonsäuremetabolismus aus, andererseits zeigen sie eine antagonistische Wirkung bezüglich einiger durch PAF (Platelet Activating Factor) geregelter physiologischer Vorgänge. Die erfindungsgemäßen Verbindungen haben daher bzw. darüber hinaus eine starke antithrombotische sowie antiatherosklerotische und antirheumatische Aktivität. Daneben zeigen die Verbindungen der allgemeinen Formel I eine günstige Beeinflussung asthmatischer Beschwerden. Sie lassen sich insbesondere zur Behandlung von entzündlichen, atherosklerotischen bzw. thromboembolischen Erkrankungen, insbesondere beim Menschen, nutzen.

Die Darstellung der erfindungsgemäßen Substanzen erfolgt durch Umsetzung einer 4-{5-[ ω-(1-Imidazolyl)-alkyl]-thien-2-yl}-4-oxo-buttersäure oder einem Ester der allgemeinen Formel II mit Hydrazin, dessen Hydrat oder Salzen, wie Hvdrochlorid, Hydrosulfat u.a., in wässrigen, wässrig-alkoholischen, alkoholischen Medien oder in indifferenten organischen Lösungsmitteln, wie z.B. Toluol bzw. deren Mischungen mit Wasser oder Alkohol bei Temperaturen von 0-150° C, vorzugsweise in Ethanol oder Wasser. Die Reaktion kann ggfs. durch Säuren, die in Form ihrer Hydraziniumsalze eingesetzt werden können, oder durch Basen, wie z.B. Erdalkalioxide, katalysiert werden.

Die Umsetzung wird durch folgende Reaktionsgleichung veranschaulicht:

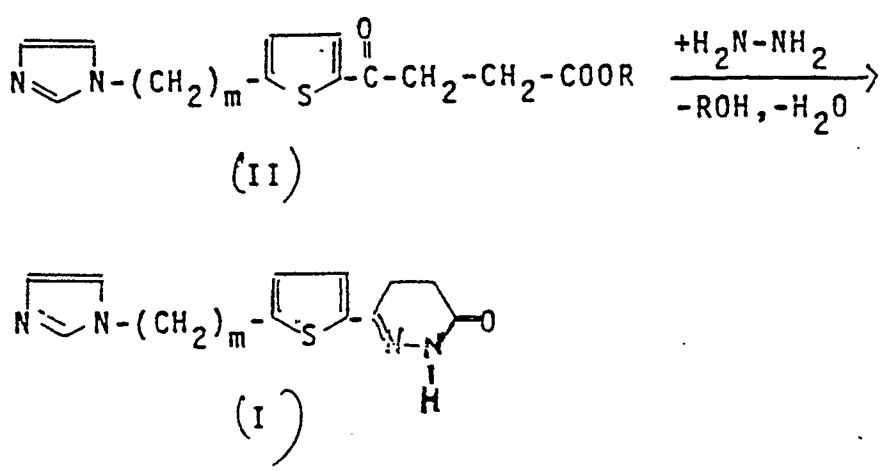


(II)

(I)

Als Ausgangsverbindungen der allgemeinen Formel II kommen insbesondere in Frage:

4-[5-(1-Imidazolylmethyl)-thien-2yl]-4-oxo-buttersäure sowie deren $C_{1-6}$-Alkylester,

4-{5-[2-(1-Imidazolyl)-ethyl-]-thien-2-yl}-4-oxo-buttersäure sowie deren $C_{1-8}$-Alkylester,
4-{5-[3-(1-Imidazolyl)-propyl]-thien-2-yl}-4-oxo-buttersäure sowie deren $C_{1-8}$-Alkylester,
4-{5-[4-(1-Imidazolyl)-butyl]-thien-2-yl}-4-oxo-buttersäure sowie deren $C_{1-6}$-Alkylester,
4-{5-[5-(1-Imidazolyl)-pentyl-]-thien-2-yl} -4-oxo-buttersäure sowie deren $C_{1-6}$-Alkylester,
4-{5-[-6-(1-Imidazolyl)-hexyl]-thien-2-yl}-4-oxo -buttersäure sowie deren $C_{1-6}$-Alkylester,
4-{5-[7-(1-Imidazolyl)-heptyl-]-thien-2-yl}-4-oxo-buttersäure sowie deren $C_{1-8}$-Alkylester,
4-{5-[8-(1-Imidazolyl)-octyl-]-thien-2-yl}-4-oxo-buttersäure sowie deren $C_{1-6}$-Alkylester,
4-{5-[9-(1-Imidazolyl)-nonyl] -thien-2-yl}-4-oxo-buttersäure sowie deren $C_{1-6}$-Alkylester,
4-{5-[10-(1-Imidazolyl)-decyl] -thien-2-yl}-4-oxo-buttersäure sowie deren $C_{1-6}$-Alkylester.
4-{5-[11-(1-Imidazolyl)-undecyl]-thien-2-yl}-4-oxo-buttersäure sowie deren $C_{1-6}$-Alkylester, und
4-{5-[12-(1-Imidazolyl)-dodecyl]-thien-2-yl}-4-oxo-buttersäure sowie deren $C_{1-6}$-Alkylester.

Die Darstellung der Ausgangsverbindungen der allgemeinen Formel II erfolgt nach an sich bekannten Verfahren.

1-(ω-Thienylalkyl)-imidazole werden durch Alkylierung von Imidazol mit dem entsprechenden ω-Halogenalkylthiophen ggfs. unter Zusatz eines organischen Lösungsmittels, wie z.B. Dimethylformamid, unter möglicher Verwendung einer Hilfsbase, wie z.B. Natriumhydrid, hergestellt (DT-OS 29 33 649). Die 1-(ω-Thienylalkyl)- imadazole wereden nach den dem Fachmann geläufigen Verfahren (Houben-Weyl, Methoden der organischen Chemie, Bd. 7/2a, S. 257 ff) mit Bersteinsäurealkylesterchlorid unter Zusatz eines organischen Lösungsmittel, wie z.B. 1,2-Dichlorethan, Nitrobenzol, Schwefelkohlenstoff, unter Verwendung eines Friedel-Crafts-Katalysators, wie z.B. Aluminiumchlorid, zu 4-{5-[ω -(1-Imidazolyl)-alkyl]-thien-2-yl}-4-oxo-buttersäurealkylestern umgesetzt.

Die Säureadditionssalze von Verbindungen der allgemeinen Formel I mit anorganischen oder organischen Säuren lassen sich durch Mischen der zugrundeliegenden Imidazolylverbindungen mit den entsprechenden Säuren in wässrigen, wässrig-organischen (z.B. Alkohol-Wasser) oder organischen Medien, wie z. B. Alkoholen, Alkohol-Ether-Mischungen oder Ether-Petrolether-Mischungen bei Temperaturen zwischen 0 und 100° C herstellen.

Die Erfindung betrifft ebenfalls pharmazeutische Präparate, welche Verbindungen der allgemeinen Formel I oder pharmazeutisch verwendbare Säureadditionssalze dieser Verbindungen enthalten. Bei der erfindungsgemäßen pharmazeutischen Präparaten handelt es sich um solche zur enteralen, wie oralen oder rektalen sowie parenteralen Verabreichung, welche die pharmazeutischen Wirkstoffe allein oder zusammen mit einem üblichen, pharmazeutisch anwendbaren Trägermaterial enthalten. Vorteilhafterweise liegt die pharmazeutische Zubereitung des Wirkstoffes in Form von Einzeldosen vor, die auf die gewünschte Verabreichung abgestimmt sind, wie z.B. Tabletten, Dragées, Kapseln, Suppositorien, Granulate, Lösungen, Emulsionen oder Suspensionen.

Die Dosierung der Verbindung liegt üblicherweise zwischen 1-500 mg pro Dosis, vorzugsweise zwischen 5-150 mg je Dosis und kann ein- oder mehrmals, bevorzugt zwei- bis dreimal täglich, verabreicht werden. Die Herstellung der erfindungsgemäßen Verbindungen wird durch die folgenden Beispiele näher erläutert. Die angegebenen Schmelzpunkte wurden mit einem Büchi 510-Schmelzpunktbestimmungsapparat gemessen und sind mit °C angegeben und nicht korrigiert. Die IR-Spektren wurden mit dem Gerät Perkin-Elmer 257 und die Massenspektren mit dem Gerät Varian MAT-311-A (70eV) aufgenommen.

**Beispiel 1**

6-[5-(1-Imidazolylmethyl)-thien-2-yl]-3-oxo-2,3,4,5-tetrahydro-pyridazin.

a) aus 4-[5-(1-Imidazolylmethyl)-thien-2-yl]-4-oxo-buttersäure-methylester.

Eine Mischung aus 7,3g Ester, 0,2g Bariumoxid, 1,3g Hydrazinhydrat und 30 ml Ethanol wird 10 Minuten bei 0°C gerührt, anschließend 20 Stunden bei Raumtemperatur und danach 4 Stunden unter Rückfluß erhitzt. Nach dem Abkühlen wird das Lösungsmittel abgezogen, der Rückstand in Wasser aufgenommen und mit Chloroform extrahiert. Die Chloroform-Phase wird mit Wasser gewaschen, über $Na_2SO_4$ getrocknet und eingeengt. Der Rückstand wird durch mehrmaliges Umkristallisieren aus Ethanol gereinigt.

Ausbeute: 2,72g, Fp. 188°

IR (in KBr): 1870 cm $^{-1}$

MS [m/e]: 280 (M+, 22%), 193 (100%), 151 (15%), 122 (39%)

b) aus 4-[5-(1-Imidazolylmethyl)-thien-2-yl] -4-oxo-buttersäure.

15 g Säure werden in 50 ml Wasser suspendiert, 3,4 g Hydrazinhydrat hinzugefügt und die Mischung 2 Stunden bei 90°C gerührt. Nach dem Abkühlen wird mit Chloroform extrahiert, die Chloroform-Phase mit Wasser gewaschen, über $Na_2SO_4$ getrocknet und bis zur Trockne eingeengt.

Ausbeute:10,9g, Fp. 187-188°C

**Darstellung der Ausgangsverbindung**

4-[5-(1-Imidazolylmethyl)-thien-2-yl]-4-oxo-buttersäuremethyl-ester.

Zu einer Suspension von 53,5g Aluminiumchlorid in 240 ml 1,2-Dichlorethan werden unter Eiskühlung 19,5g Bernsteinsäuremethylesterchlorid und anschließend eine Lösung von 20g 1-(Thien-2-ylmethyl)-imidazol in 200 ml 1,2-Dichlorethan zugetropft. Danach wird die Mischung 3 Stunden bei 50°C gerührt. Nach dem Abkühlen wird die Reaktionsmischung in eine Mischung aus 147,3g Ethylendiamintetraessigsäure und 500g Eis eingerührt und durch Zugabe von verdünnter Natronlauge auf ca.pH 8 gebracht. Die Phasen werden getrennt, die organischt Phase über $Na_2SO_4$ getrocknet und eingeengt. Durch Ausrühren des Rückstandes mit Hexan erhält man farblose Kristalle.

Ausbeute: 26,3g, Fp. 69°-70°C

IR (in KBr): 1720, 1655 $cm^{1}$

MS [m/e]: 278 (M+ 32%), 247 (17%), 211 (100%)

4-[5-(1-Imidazolylmethyl)-thien-2-yl]-4-oxo-buttersäure. Eine Mischung aus 4,17g 4-[5-(1-Imidazolylmethyl)-thien-2-yl-]-4-oxo-buttersäuremethylester, 0,8 g Natriumhydroxid und 20 ml Methanol wird 8 Stunden bei Raumtemperatur gerührt. Anschließend wird das Lösungsmittel abgezogen und der Rückstand in Wasser aufgenommen. Die Lösung wird mehrmals mit Chloroform extrahiert, die Chloroform-Phase verworfen. Die wässrige Lösung wird mit verdünnter Salzsäure auf ca. pH 7 gebracht und bis zur Trockne eingeengt. Der Rückstand wird durch Säulenchromatographie (Kieselgel//n$CHCl_3$/Methanol) gereinigt.

Ausbeute: 1,4g, Fp.203°-205°C (Z)

IR (in KBr): 1700, 1860 $cm^{-1}$

M5 [m/e]: 264 (M+, 1,5%), 220 (6%), 197 (100%), 192 (27%), 151 (40%), 124 (28%), 97 (41%)

**Beispiel 2**

6-{5-[2-(1-Imidazolyl)-ethyl]-thien-2-yl}-3-oxo-2,3,4,5-tetrahydro-pyridazin.

4,2g 4-{5-[2-(1-Imidazolyl)-ethyli-thien-2-yl}-4-oxo-buttersäure werden in 10 ml Wasser suspendiert, 0,8g Hydrazinhydrat hinzugefügt und die Mischung 2 Stunden bei 90°C gerührt. Nach dem Abkühlen wird mit Chloroform extrahiert. Die Chloroform-Phase wird mit Wasser gewaschen, über $Na_2SO_4$ getrocknet und zur Trockne eingeengt.

Ausbeute: 3,3g, Fp. 137°C

IR (in KBr): 1670 cm

MS [m/e]: 274 (M+, 52%), 246 (3%), 206 (9%), 193 (100%), 151 (21%), 135 (9%), 122 (18%)

**Beispiel 3**

6-{5-[5-(1-Imidazolyl)-pentyl-]-thien-2-yl -3-oxo-2,3,4,5-tetrahydro-pyridazin.

3,2g 4-{5-[S-(1-Imidazolyl)-pentyl]-thien-2-yl} -4-oxo-butter-säure werden in 10 ml Wasser suspendiert, 0,52g Hydrazinhydrat hinzugefügt und die Mischung 2 Stunden bei 90°C gerührt. Nach dem Abkühlen wird der entstandene Niederschlag abgesaugt, mit Wasser gewaschen und getrocknet.

Ausbeute: 2,5g, Fp. 114°C

**Beispiel 4**

6-{5-[8-(1-Imidazolyl)-octyl] -thien-2-yl} -3-oxo-2,3,4,5-tetrahydro-pyridazin.

1,8g 4-{5-[8-(1-Imidazolyl)-octyl]-thien-2-yl}-4-oxo-buttersäure werden in 10 ml Wasser suspendiert, 0,26g Hydrazinhydrat hinzugefügt und die Mischung 2 Stunden bei 90°C gerührt. Nach Abkühlen wird der entstandene Niederschlag abgesaugt, mit Wasser gewaschen und getrocknet.

Ausbeute: 0,6g, Fp. 103-105°C

IR (in KBr): 1690 $cm^{-1}$

MS [m/e]: 358 (M+, 100%), 325 (69%), 288 (92%), 207 (11%), 193 (33%), 179 (17%), 165 (16%), 151 (34%), 137 (25%), 123 (33%), 109 (43%).

Analog den Beispiel 1-4 werden hergestellt:

5. 6-{5-[3-(1-Imidazolyl)-propyl]-thien-2-yl{-3-oxo-2,3,4,5-tetrahydro-pyridazin,
6. 6-{5-[4-(1-Imidazolyl)-butyl]-thien-2-yl}-3-oxo-2,3,4,5-tetrahydro-pyridazin,
7. 8-{5-[8-(1-Imidazolyl)-hexyl]-thien-2-yl}-3-oxo-2,3,4,5-tetrahydro-pyridazin,
8. 8-{5-[7-(1-Imidazolyl)-heptyl]-thien-2-yl}-3-oxo-2,3,4,5-tetrahydro-pyridazin,
9. 6-{5-[8-(1-Imidazolyl)-octyl-]-thien-2-yl}-3-oxo-2,3,4,5-tetrahydro-pyridazin,
10. 6-{5-[9-(1-Imidazolyl)-nonyl] -thien-2-yl}-3-oxo-2,3,4,5-tetrahydro-pyridazin,

11. 8-{5-[10-(1-Imidazolyl)-decyl]-thien-2-yl}-3-oxo-2,3,4,5-tetrahydro-pyridazin,
12. 8-{5-[11-(1-Imidazolyl)-undecyl] -thien-2-yl}-3-oxo-2,3,4,5-tetrahydro-pyridazin
13. 8-{5-[12-(1-Imidazolyl)-dodecyl]-thien-2-yl}-3-oxo-2,3,4,5-tetrahydro-pyridazin.

**Beispiel 14**

6-[5-(l-Imidazolylmethyl)-thien-2-yl-]-3-oxo-2,3,4,5-tetrahy-dropyridazin-Fumarsäure-Salz.

Eine Mischung aus 1,5g 6-/5-(1-Imidazolylmethyl)-thien-2-yl[-3-oxo-2,3,4,5-tetrahydro-pyridazin und .0,66g Fumarsäure wird in 30 ml Ethanol ca. 30 Minuten unter Rückfluß erhitzt, bis sich eine klare Lösung gebildet hat. Beim Abkühlen kristallisiert das Salz aus, das abgesaugt und getrocknet wird.

Ausbeute: 1,6 g, Fp. 182°C

IR (in KBr): 1670 $cm^{-1}$

Analog Beispiel 14 lassen sich z.B. Oxalate, Succiniate oder Malonate sowie anorganische Salze, wie Hydrochloride und Hydrosulfate herstellen.

**Patentansprüche**

für die Vertragsstaaten: BE, CH, FR, GB, IT, LI, LU, NL, SE

1. 6-{-5-[ω-(1-Imidazolyl)-alkyl]-thien-2-yl} -3-oxo-2,3,4,5-tetrahydro-pyridazine der allgemeinen Formel I

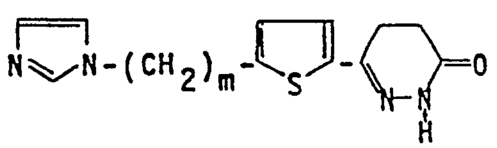

(I)

in der

m = eine ganze Zahl von 1-12 bedeutet, sowie deren Säureadditionssalze mit anorganischen oder organischen Säuren.

2. 6-[5-(1-Imidazolylmethyl)-thien-2-yl] -3-oxo-2,3,4,5-tetra-hydro-pyridazin und dessen pharmazeutisch verträgliche Säureadditionssalze.

3. 6-{5-[2-(1-Imidazolyl)-ethyl]-thien-2-yl} -3-oxo-2,3,4,5-tetrahydro-pyridazin und dessen pharmazeutisch verträgliche Säureadditionssalze.

4. 6-{5-[3-(1-Imidazolyl)-propyl] -thien-2-yl}-3-oxo-2,3,4,5-tetrahydro-pyridazin und dessen pharmazeutisch verträgliche Säureadditionssalze.

5. 6-{5-[4-(1-Imidazolyl)-butyl]-thien-2-yl}-3-oxo-2 3 4 5-tetrahydro-pyridazin und dessen pharmazeutisch verträgliche Säureadditionssalze.

8. 6-{5-[5-(1-Imidazolyl)-pentyl] -thien-2-yl}-3-oxo-2,3,4,5-tetrahydro-pyridazin und dessen pharmazeutisch verträgliche Säureadditionssalze.

7. 6-{5-[6-(1-Imidazolyl)-hexyl] -thien-2-yl}-3-oxo-2,3,4,5-tetrahydro-pyridazin und dessen pharmazeutisch verträgliche Säureadditionssalze.

8. 6-{5-[7-(1-Imidazolyl)-heptyl] -thien-2-yl}-3-oxo-2,3,4,5-tetrahydro-pyridazin und dessen pharmazeutisch verträgliche Säureadditionssalze.

9. 6-{5-[8-(1-Imidazolyl)-octyl]-thien-2-yl}-3-oxo-2,3,4,5-tetrahydro-pyridazin und dessen pharmazeutisch verträgliche Säureadditionssalze.

10. 6-{5-[9-(1-Imidazolyl)-nonyl]-thien-2-yl} -3-oxo-2,3,4,5-tetrahydro-pyridazin und dessen pharmazeutisch verträgliche Säureadditionssalze.

11. 6-{5-[10-(1-Imidazolyl)-decyl]-thien-2-yl}-3-oxo-2,3,4,5-tetrahydro-pyridazin und dessen pharmazeutisch verträgliche Säureadditionssalze.

12. 6-{5-[11-(1-Imidazolyl)-undecyl]-thien-2-yl}-3-oxo-2,3,4,5-tetrahydro-pyridazin und dessen pharmazeutisch verträgliche Säureadditionssalze.

13. 6-{5-[12-(1-Imidazolyl)-dodecyl ]-thien-2-yl}-3-oxo-2,3,4,5-tetrahydro-pyridazin und dessen pharmazeutisch verträgliche Säureadditionssalze.

14. Verfahren zur Herstellung der Verbindungen gemäß den Ansprüchen 1-13, dadurch gekennzeichnet, daß man eine 4-{5-[ω-(1-Imidazolyl)-alkyl]-thien-2-yl}-4-oxo-buttersäure oder deren Ester der allgemeinen Formel II

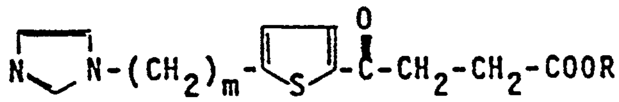

(II)

wobei m die in der allgemeinen Formel 1 angegebene Bedeutung hat und R Wasserstoff oder $C_{1-6}$-Alkyl bedeutet, mit Hydrazin, dessen Hydrat oder einem Hydrazinsalz in wäßrigen, wäßrigalkoholischen, alkoholischen Medien oder in indifferenten organischen Lösungsmitteln bzw. deren Mischungen mit Wasser oder Alkohol bei einer Temperatur im Bereich von 0-150°C, gegebenenfalls unter Zuhilfenahme eines der für Aminolysen und Kondensationsreaktionen üblichen Katalysatoren umsetzt.

15. Arzneimittelzubereitungen, dadurch gekennzeichet, daß sie eine Verbindung oder ein pharmazeutisch verträgliches Säureadditionssalz dieser Verbindung gemäß den Ansprüchen 1-13 zusammen mit einem pharmazeutisch geeigneten Verdünnungsmittel oder Trägermaterial enthalten.

**Patentanspruch**

für den Vertragsstaat: AT

Verfahren zur Herstellung von 6-{5-[ω-(1-Imidazolyl)-alkyl] -thien-2-yl}-3-oxo-2,3,4,5-tetrahydro-pyridazinen der allgemeinen Formel I

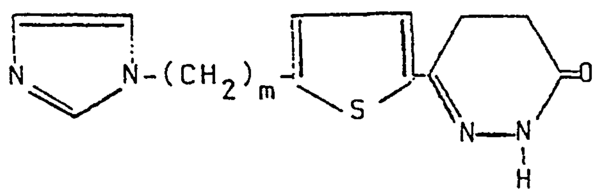

(I)

in der m eine ganze Zahl von 1 - 12 bedeutet,

sowie deren Säureadditionssalzen mit anorganischen cder organischen Säuren, dadurch gekennzeichnet, daß man eine 4-{5-[ω-(1-Imidazolyl)-alkyl]-thien-2-yl} -4-oxo-butter-säure oder deren Ester der allgemeinen Formel II

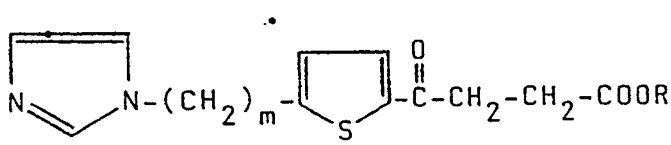

(II)

wobei m die in der allgemeinen Formel I angegebene Bedeutung hat und R Wasserstoff oder $C_{1-6}$-Alkyl bedeutet, mit Hydrazin, dessen Hydrat oder einem Hydrazinsalz in wäßrigen, wäßrig-alkoholischen, alkoholischen Medien oder in indifferenten organischen Lösungsmitteln bzw. deren Mischungen mit Wasser oder Alkohol bei einer Temperatur im Bereich von 0 - 150°C, gegebenenfalls unter Zuhilfenahme eines der für Aminolysen und Kondensationsreaktionen üblichen Katalysatoren umsetzt.

**Claims**

for the Contracting states: BE, CH, FR, GB, IT, LI, LU, NL, SE

1. 6-{5-[ω-(1-Imidazolyl)-alkyl]-thien-2-yl}-3-oxo-2,3,4,5-tetrahydropyridazine of the general formula I

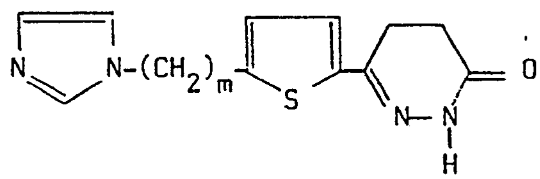

(I)

wherin m is a numeral from 1 to 12, as well as the acid addition salts with inorganic or organic acids.

2. 6-[5-(1-Imidazolylmethyl)-thien-2-yl]-3-oxo-2,3,4,5-tetrahydropyridazine and its pharmaceutically compatible salts.

3. 6-5-[32-(Imidazolyl)-ethyl]-thien-2-yl}-3-oxo-2,3,4,5 tetrahydropyridazine and its pharmaceutically compatible salts.

4. 6-5-[3-(1-Imidazolyl)-propyl]-thien-2-yl}-3-oxo-2,3,4,5-tetrahydropyridazine and its pharmaceutically compatible salts.

5. 6-5-[4-(1-Imidazolyl)-butyl]-thien-2-yl}-3-oxo-2,3,4,5-tetrahydropyridazine and its pharmaceutically compatible salts.

6. 6-{5-[5-(1-Imidazolyl)-pentyl]-thien-2-yl} -3-oxo-2,3,4,5-tetrahydropyridazine and its pharmaceutically compatible acid addition salts.

7. 6-{5-[6-(1-Imidazolyl)-hexyl-]-thien-2-y}-3-oxo-2,3,4,5-tetrahydropyridazine and its pharmaceutically compatible acid addition salts.

8. 6-{5-[7-(1-Imidazolyl)-heptyl]-thien-2-yl}-3-oxo-2,3,4,5-tetrahydropyridazine and its pharmaceutically compatible acid addition salts.

9. 6-{5-[8-(1-Imidazolyl)-octyl]-thien-2-yl}-3-oxo-2,3,4,5-tetrahydropyridazine and its pharmaceutically compatible acid addition salts.

10. 6-{5-[9-(1-Imidazolyl)-nonyl]-thien-2-yl} -3-oxo-2,3,4,5-tetrahydropyridazine and its pharmaceutically compatible acid addition salts.

11. 6-{5-[10-(1-Imidazolyl)-decyl]-thien-2-yl} -3-oxo-2,3,4,5-tetrahydropyridazine and its pharmaceutically compatible acid addition salts.

12. 6-{5-[11-(1-Imidazolyl)-undecyl]-thien-2-yl}-3-oxo-2,3,4,5-tetrahydropyridazine and its pharmaceutically compatible acid addition salts.

13. 6- {5-[12-(1-Imidazolyl)-dodecyl]-thien-2-yl} -3-oxo-2,3,4,5-tetrahydropyridazine and its pharmaceutically compatible acid addition salts.

14. Process for the preparation of compounds according to claims 1 to 13, characterised in that a 4-{5-[-ω-(1-Imidazolyl)-alkyl]-thien-2-yl} -4-oxo-butyric acid or an ester thereof having the general formula II

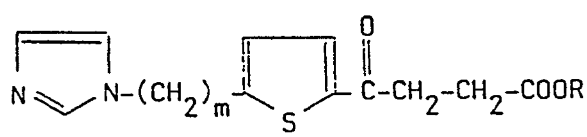

(II)

wherein m has the meaning as given in formula I and R is hydrogen or a $C_{1-6}$-alkyl group, is subjected to reaction with hydrazine, its hydrate or a salt of hydrazine, in an aqueous, aqueous-alcoholic or alcoholic reaction mixture or in an inert organic solvent or its mixtures with water or alcohol at a temperatur of from 0 to 150° C, possibly in the presence of a catalyst usual for ammolytic acid condensation reactions.

15. Pharmaceutical preparations characterised in that they contain a compound or a pharmaceutically compatible acid addition salt thereof as claimed in claims 1 to 13 together with pharmaceutically suitable diluents or carrier materials.

**Claim**

for the Contracting state: AT

Process for the preparation of 6-{5-[ω-(1-Imidazolyl)-alkyl]-thien-2-yl}-3-oxo-2,3,4,5-tetrahydro-pyridazines of the general formula I

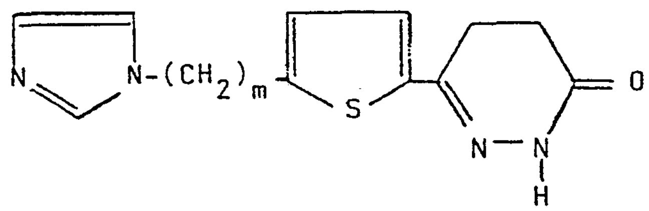

(I)

wherein m is a numeral of from 1 to 12, as well as the acid addition salts thereof with inorganic and organic acids, characterised in that a 4-{5-[ω-(1-Imidazolyl)-alkyl]-thien-2-yl} -4-oxo-butyric acid or an ester thereof having the general formula II

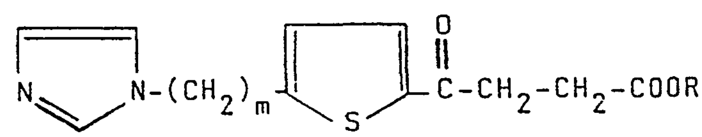

(II)

wherein m has the meaning given for general formula I and R is hydrogen or a $C_{1-6}$-alkyl group, is subjected to reaction with hydrazine, its hydrate or a salt thereof in an aqueous, aqueous-alcoholic or alcoholic reaction medium or in an inert organic solvent or mixtures thereof with water or alcohol at a temperature of from 0 - 150° C, possibly in the presence of a catalyst usual for aminolysis and condensation reactions.

**Revendications**

pour les Etats contractants BE, CH, FR, GB, IT, LI, LU, NL, SE

1 - 6- { 5-[-ω -(1-imidazolyl)-alcoyl]-thién-2-yl} -3-oxo-2,3,4,5-tétrahydro-pyridazines de formule générale I

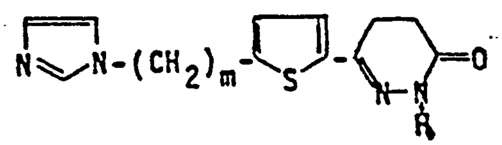

(I)

où

m représente un nombre entier allant de 1 à 12, ainsi que leurs sels d'addition d'acides avec des acides inorganiques ou organiques.

2 - 6-[5-(1-imidazolylméthyl)-thién-2-yl]-3-oxo-2,3,4,5-tétrahydro-pyridazine et ses sels d'addition d'acides pharmaceutiquement acceptables.

3 - 6-{5-[2-(1-imidazolyl)-éthyl]-thién-2-yl} -3-oxo-2,3,4,5-tétrahydro-pyridazine et ses sels d'addition d'acides pharmaceutiquement acceptables.

4 - 6-{5-[3-(1-imidazolyl)-propyl]-thién-2-yl} -3-oxo-2,3,4,5-tétrahydro-pyridazine et ses sels d'addition d'acides pharmaceutiquement acceptables.

5 - 6-{5-[4-(1-imidazolyl)-butyl]-thién-2-yl} -3-oxo-2,3,4,5-tétrahydro-pyridazine et ses sels d'addition d'acides pharmaceutiquement acceptables.

6 - 6-{5-[5-(1-imidazolyl)-pentyl]-thién-2-yl} -3-oxo-2,3,4,5-tétrahydro-pyridazine et ses sels d'addition d'acides pharmaceutiquement acceptables.

7 - 6-{5-[6-(1-imidazolyl)-hexyl]-thién-2-yl} -3-oxo-2,3,4,5-tétrahydro-pyridazine et ses sels d'addition d'acides pharmaceutiquement acceptables.

8 - 6-{ 5-[7-(1-imidazclyl)-heptyl] -thién-2-yl} -3-oxo-2,3,4,5-tétrahydro-pyridazine et ses sels d'addition d'acides pharmaceutiquement acceptables.

9 - 6-{5-[8-(1-imidazolyl)-octyl]-thién-2-yl} -3-oxo-2,3,4,5-tétrahydro-pyridazine et ses sels d'addition d'acides pharmaceutiquement acceptables.

10 - 6-{5-[9-(1-imidazolyl)-nonyl] -thién-2-yl} -3-oxo-2,3,4,5-tétrahydro-pyridazine et ses sels d'addition d'acides pharmaceutiquement acceptables.

11 - 6-{5-[10-(1-imidazolyl)-décyl]-thién-2-yl{ -3-oxo-2,3,4,5-tétrahydro-pyridazine et ses sels d'addition d'acides pharmaceutiquement acceptables.

12 - 6-{5-[11-(1-imidazolyl)-undécyl] -thién-2-yl}-3-oxo-2,3,4,5-tétrahydro-pyridazine et ses sels d'addition d'acides pharmaceutiquement acceptables.

13 - 6-{5-[12-(1-imidazolyl)-dodécyl] -thién-2-yl} d'acides pharmaceutiquement acceptables.

14 - Procédé de préparation des composés selon les revendications 1-13, caractérisé en ce qu'on fait réagir un acide 4-{5-[ω-(1-imidazolyl)-alcoyl]-thién-2-yl}-4-oxo-butyrique ou un ester de celui-ci de formule générale II

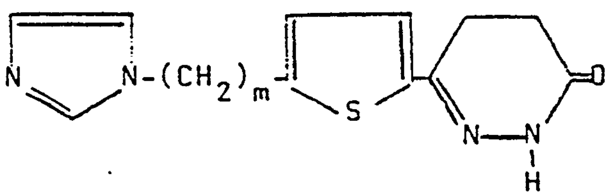

(I)

où m a la signification donnée dans la formule générale I et R représente un hydrogène ou un alcoyle en C1 à C6, avec de l'hydrazine, son hydrate ou un sel d'hydrazine dans des milieux aqueux, hydroalcooliques, alcooliques ou dans des solvants organiques indifférents ou selon les cas leurs mélanges avec de l'eau ou de l'alcool à une température située dans un intervalle de 0-150°C, éventuellement à l'aide d'un des catalyseurs habituels pour les aminolyses et les réactions de condensation.

15 - Préparations médicamenteuses, caractérisées en ce qu'elles contiennent un composé ou un sel d'addition d'acide pharmaceutiquement acceptable de ce composé selon les revendications 1-13 avec un diluant ou support pharmaceutique approprié.

**Revendication**

pour l'Etat contractant: AT

Procédé de préparation de 6-{5-[ω-(1-imidazolyl)-alcoyl]-thién-2-yl}-3-oxo-2,3,4,5-tétrahydro-pyridazines de formule générale 1

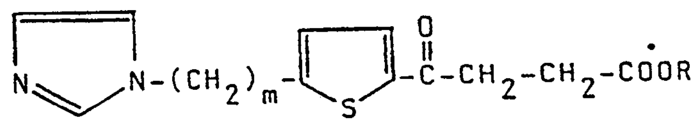

(II)

où

m représente un nombre entier allant de 1 à 12, ainsi que leurs sels d'addition d'acides avec des acides inorganiques ou organiques et caractérisé en ce qu'on fait réagir un acide 4-{5-[ω-(1-imidazolyl)-alcoyl]-thién-2-yl} -4-oxo-butyrique ou un ester de celui-ci de formule générale II

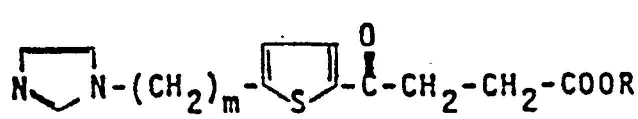

(II)

où m a la signification donnée dans la formule générale I et R représente un hydrogène ou un alcoyle en Cl à C6, avec de l'hydrazine, son hydrate ou un sel d'hydrazine dans des milieux aqueux, hydroalcooliques,

alcooliques ou dans des solvants organiques indifférents ou selon les cas leurs mélanges avec de l'eau ou de l'alcool à une température située dans un intervalle de 0-150°C, éventuellement à l'aide d'un des catalyseurs habituels pour les aminolyses et les réactions de condensation.